# EUROPEAN PATENT SPECIFICATION

(11) **EP 4 192 422 B1**
(45) Date of publication and mention of the grant of the patent: **11.06.2025**
(21) Application number: 20841891.3
(22) Date of filing: 16.12.2020
(51) Int. Cl.: A61K 8/49, A61K 8/42, A61K 8/365, A61Q 11/00

(54) **COOLING SENSATION COMPOSITIONS**
KÜHLEMPFINDUNGS-ZUSAMMENSETZUNGEN
COMPOSITIONS DE SENSATION DE REFROIDISSEMENT

(30) Priority: 05.08.2020 GB 202012170
(43) Date of publication of application: 14.06.2023
(73) Proprietor: Givaudan SA, 1214 Vernier (CH)
(72) Inventor: SAJI, Norikazu, Canterbury Kent CT4 7TA (GB); BARTHOLOMEW, Tracy, Ashford Kent TN25 0UP (GB); COCITO ARMANINO, Nicolas, 5400 Baden (CH)
(74) Representative: Global Patents
(86) International application number: PCT/EP2020/086367
(87) International publication number: WO 2022/028726

(56) References cited:
- WO-A2-2009/051632
- DE-A1- 102008 015 428
- US-A- 4 190 643

## Description

### TECHNICAL FIELD

This invention is concerned with compositions, and especially liquid composition comprising at least one non-natural cooling compound and lactic acid, and the use of said liquid composition in a flavour or fragrance formulation, wherein, for the purpose of defining the invention, the combination of non natural cooling compound and lactic acid is limited in the sense of claim 1 to the specific list of cooling compounds cited therein.

### BACKGROUND

Compounds providing a cooling sensation have for a long time played an important role in the flavor and fragrance industry in order to produce an association with freshness and cleanliness. Cooling compounds are widely used in a variety of products such as foodstuffs, tobacco products, beverages, dentifrices, mouthwashes, toothpastes, and toiletries. The cooling sensation provided contributed to the appeal and acceptability of consumer products. In particular, oral care products, such as dentifrices and mouthwashes are formulated with coolants because they provide breath freshening effects and a clean, cool, fresh feeling in the mouth.

The newest generation of non-natural cooling compounds is very potent at low concentrations, but unfortunately a lot of them are at room temperature crystalline solids and the use is not without problems. Firstly, it is not always convenient or easy to mix solids into consumer products, which may be in a liquid or a paste-like form. Secondly, powder-like ingredients must be handled with caution to avoid dust hazards associated therewith. See e.g. DE102008015428, US4190642 and WO2009/051632.

The use of ethyl lactate as a solvent for potent cooling compounds, in particular N-(1H-pyrazol-3-yl)-N-(thiophen-2-ylmethyl)-2-(p-tolyloxy)acetamide is disclosed in WO2019012071. Ethyl lactate (CAS 97-64-3) is known to possess fruity odor and taste. Accordingly, there remains a need to provide cooling compounds in a form that is easy to use in further formulation operations, which remains stable in that form for a long period of storage, and which essentially do not contribute to the overall flavour / fragrance impression of flavour / fragrance formulations..

### SUMMARY OF THE INVENTION

In accordance with a first aspect of the present invention there is provided a liquid composition comprising, consisting essentially of, or consisting of at least one non-natural cooling compound and lactic acid. acid, wherein, for the purpose of defining the invention, the combination of non natural cooling compound and lactic acid is limited in the sense of claim 1 to the specific list of cooling compounds cited therein.

In accordance with a second aspect of the present invention there is provided a fragrance or flavour formulation comprising a liquid composition of the first aspect of the present invention and at least one active selected from the group consisting of flavour and fragrance.

In accordance with a third aspect of the present invention there is provided a fragranced or flavoured product comprising a liquid composition of the first aspect of the present invention or a formulation of the second aspect of the present invention, and a product base.

In certain embodiments, the product is selected from consumer products which get into contact with the human skin and/or mucosa, including food products, beverages, chewing gum, tobacco and tobacco replacement products, dental care products, personal care products, including lip care products, sexual health and intimate care products.

In certain embodiments, the product is selected from air care products, such as an air freshener or a "ready to use" powdered air freshener which can be used in the home space (rooms, refrigerators, cupboards, shoes or car) and/or in a public space (halls, hotels, malls, etc .. ).

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### DETAILED DESCRIPTION

The present invention is based, at least in part, on the surprising finding that non-natural cooling compounds dissolve in higher quantities in lactic acid.

Thus there is provided in a first aspect a liquid composition comprising, consisting essentially of, or consisting of
a) at least one non-natural cooling compound , and
b) lactic acid, wherein, for the purpose of defining the invention, the combination of non natural cooling compound and lactic acid is limited in the sense of claim 1 to the specific list of cooling compounds cited therein.

Applicant surprisingly found that non-natural cooling compounds are particular well soluble in lactic acid.

By "particular well soluble" it is meant within the context of the invention that a stable liquid composition comprising up to 50 weight % of a non-natural cooling compound can be prepared. Even though compositions with higher concentrations may be prepared, compositions comprising lower concentrations (e.g. 30 weight % or less, such as 25, 20, 15, 10, or 5 weight % or less) are preferred due to the viscosity of the resulting composition. The more viscous a liquid is, the more difficult it is to dose.

An advantage of such highly concentrated liquid compositions is that the contribution of an intrinsic odour of the used solvent can be minimised. This is particularly important when such cooling compositions are used in combination with flavour and fragrance formulations. It is also worthwhile to note that the admixture of liquids, e.g., to a flavour / fragrance formulation could be regarded more sustainable than admixing a solid, which would require more energy.

By "stable" it is meant within the context of the invention that no precipitation was observed when stored for up to four weeks at a temperature of at least room temperature (i.e. about 22°C).

By "non-natural cooling compounds" it is meant compounds selected from the group consisting of 2-methyl-1-(2-(5-(p-tolyl)-1H-imidazol-2-yl)piperidin-1-yl)butan-1-one (including (2S)-2-methyl-1-(2-(5-(p-tolyl)-1H-imidazol-2-yl)piperidin-1-yl)butan-1-one, and (2R)-2-methyl-1-(2-(5-(p-tolyl)-1H-imidazol-2-yl)piperidin-1-yl)butan-1-one), 2-(methylthio)-1-(2-(5-(p-tolyl)-1H-imidazol-2-yl)piperidin-1-yl)propan-1-one, 2-methyl-2-(methylthio)-1-(2-(5-(p-tolyl)-1H-imidazol-2-yl)piperidin-1-yl)propan-1-one, 2,2-dimethyl-1-(2-(5-(p-tolyl)-1H-imidazol-2-yl)piperidin-1-yl)but-3-en-1-one, N-(1H-pyrazol-3-yl)-N-(thiophen-2-ylmethyl)-2-(p-tolyloxy)acetamide, 2-(4-ethylphenoxy)-N-(1H-pyrazol-3-yl)-N-(thiophen-2-ylmethyl)acetamide, *N*-Ethyl-*p*-menthane-3-carboxamide, ethyl (2-isopropyl-5-methylcyclohexane-1-carbonyl)glycinate, 2-isopropyl-N,2,3-trimethylbutanamide, N-(4-(2-amino-2-oxoethyl)phenyl)-2-isopropyl-5-methylcyclohexane-1-carboxamide (including *rac*-(1R,2S,5R)-N-(4-(2-amino-2-oxoethyl)phenyl)-2-isopropyl-5-methylcyclohexane-1-carboxamide), 2-isopropyl-5-methylcyclohexyl 2-hydroxypropanoate, N-(2-hydroxy-2-phenylethyl)-2-isopropyl-5,5-dimethylcyclohexane-1-carboxamide, and mixtures thereof.

In one particular embodiment the liquid composition comprises at least 1 weight % (e.g. 2 - 50 weight % (e.g., 2.5 - 20 weight %) of a non-natural cooling compound.

Lactic acid is chiral, consisting of two enantiomers. One is known as I-(+)-lactic acid or (S)-lactic acid and the other, its mirror image, is d-(-)-lactic acid or (R)-lactic acid. A mixture of the two in equal amounts is called dl-lactic acid, or racemic lactic acid. dl-Lactic acid is miscible with water and with ethanol above its melting point, which is around 17 °C. Lactic acid is hygroscopic and thus in the food and beverage industry quite often used with a purity of about 85 - 90 weight %. L-Lactic acid is supplemented into foods and beverages (E270), and is widely used as a non-volatile acidulant. The use of the term "lactic acid" in this description encompasses not only the individual enantiomers and the racemate in their pure forms, but also the commercially-available forms of lactic acid, which are generally 85-90 wt % pure.

Lactic acid is commercially available, e.g., from PURAC Biochem BV (The Netherlands), and Prinova Europe LTD.

The liquid composition as defined hereinabove may be added to any products in which a cooling effect on the skin or mucous membrane is desired. It may be employed in the product simply by directly mixing the liquid composition with the product, or it may, in an earlier step, be entrapped in a suitable entrapment material. Alternatively the liquid composition as hereinabove defined may be admixed with other actives, such as, flavours, fragrances, and sweetening agents, and mixtures thereof, before employing it into the product.

Thus there is provided in a further embodiment a flavour or fragrance formulation comprising a liquid composition comprising, consisting essentially of, or consisting of at least one non-natural cooling compound , and lactic acid, and wherein the formulation further comprises at least one active selected from the group consisting of flavour and fragrance, and optionally comprising sweetening agent.

Examples of flavour ingredients include natural flavors, artificial flavors, spices, seasonings, and the like. Exemplary flavor ingredients include synthetic flavor oils and flavoring aromatics and/or oils, oleoresins, essences, and distillates, and a combination comprising at least one of the foregoing.

Flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil; useful flavoring agents include artificial, natural and synthetic fruit flavors such as vanilla, and citrus oils including lemon, orange, lime, grapefruit, yuzu, sudachi, and fruit essences including apple, pear, peach, grape, raspberry, blackberry, gooseberry, blueberry, strawberry, cherry, plum, prune, raisin, cola, guarana, neroli, pineapple, apricot, banana, melon, apricot, cherry, tropical fruit, mango, mangosteen, pomegranate, papaya, and so forth.

Additional exemplary flavors imparted by a flavoring composition include a milk flavor, a butter flavor, a cheese flavor, a cream flavor, and a yogurt flavor; a vanilla flavor; tea or coffee flavors, such as a green tea flavor, an oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a chamomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, a wintergreen flavour, and a wasabi (Japanese horseradish) flavor; a nut flavor such as an almond flavor, a hazelnut flavor, a macadamia nut flavor, a peanut flavor, a pecan flavor, a pistachio flavor, and a walnut flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor.

Generally any flavoring or food additive (including food colors) such as those described in "Essential guide to food additives", Third edition2008, page 101 - 321 (ISBN: 978-1-905224-50-0) by Leatherhead Food International Ltd., can be used. The publication is incorporated herein by reference.

In one particular embodiment the at least one active may be selected from anethole, menthol laevo, carvone laevo, ethyl maltol, vanillin, eucalyptol, eugenol, menthol racemic, cis-3-hexenol, linalol, mint oil (e.g. peppermint arvensis oil, peppermint piperita oil, spearmint native oil, spearmint scotch oil), corylone, ethyl butyrate, cis-3-hexenyl acetate, citral, eucalyptus oil, ethyl-vanillin, methyl salicylate, 2'-hydroxypropiophenone, ethyl acetate, methyl dihydro jasmonate, geraniol, lemon oil, iso amyl acetate, thymol, ionone beta, linalyl acetate, decanal, cis jasmone, ethyl hexanoate, melonal (2,6-dimethylhept-5-enal), citronellol, ethyl aceto acetate, nutmeg oil and clove oil, or mixtures thereof.

Examples of sweetening agents include, but are not limited to, sucrose, fructose, glucose, high fructose corn syrup, corn syrup, xylose, arabinose, rhamnose, erythritol, xylitol, mannitol, sorbitol, inositol, acesulfame potassium, aspartame, neotame, sucralose, and saccharine, and mixtures thereof; trilobatin, hesperetin dihydrochalcone glucoside, naringin dihydrochalcone, mogroside V, Luo Han Guo extract, rubusoside, rubus extract, glycyphyllin, isomogroside V, mogroside IV, siamenoside I, neomogroside, mukurozioside IIb, (+)-hernandulcin, 4 β -hydroxyhernandulcin, baiyunoside, phlomisoside I, bryodulcoside, bryoside bryonoside, abrusosides A-E, cyclocarioside A, cyclocaryoside I, albiziasaponins A-E, glycyrrhizin, araboglycyrrhizin, periandrins I-V, pterocaryosides A and B, osladin, polypodosides A and B, telosmoside A8-18, phyllodulcin, huangqioside E neoastilbin, monatin, 3-acetoxy-5,7-dihydroxy-4'-methoxyflavanone, 2R,3R-(+)-3-Acetoxy-5,7,4'-trihydroxyflavanone, (2R,3R)-dihydroquercetin 3-O-acetate, dihydroquercetin 3-O-acetate 4'-methyl ether, brazzein, curculin, mabinlin, monellin, neoculin, pentadin, thaumatin, and combinations thereof. Some of the compounds listed above are known sweetness enhancers as well as sweeteners. When used as sweetness enhancers they are normally used below their sweetness detection thresholds.

In a further aspect there is provided a flavoured or perfumed product comprising a flavour or fragrance formulation and a product base, e.g., an orally acceptable carrier for products which are taken into the mouth, and skin tolerable carriers for products which get into contact with the skin.

In some aspects, the orally acceptable carrier may comprise one or more compatible solid or liquid excipients or diluents which are suitable for topical oral administration. By "compatible," as used herein, is meant that the components of the composition are capable of being commingled without interaction in a manner which would substantially reduce stability and/or efficacy. The carriers can include the usual and conventional components of dentifrices, non-abrasive gels, subgingival gels, mouthwashes or rinses, mouth sprays, chewing gums, lozenges and breath mints. The choice of a carrier to be used is basically determined by the way the composition is to be introduced into the oral cavity. Carrier materials for toothpaste, tooth gel or the like include abrasive materials, sudsing agents, binders, humectants, flavoring and sweetening agents, etc. as disclosed in e.g., U.S. Pat. No. 3,988,433, to Benedict. Carrier materials for biphasic dentifrice formulations are disclosed in U.S. Pat. Nos. 5,213,790; 5,145,666 and 5,281,410 all to Lukacovic et al., and in U. S. Patents 4,849,213 and 4,528,180 to Schaeffer. Mouthwash, rinse or mouth spray carrier materials typically include water, flavoring and sweetening agents, etc., as disclosed in, e.g., U.S. Pat. No. 3,988,433 to Benedict. Lozenge carrier materials typically include a candy base; chewing gum carrier materials include a gum base, flavoring and sweetening agents, as in, e.g., U.S. Pat. No. 4,083,955, to Grabenstetter et al.. Sachet carrier materials typically include a sachet bag, flavoring and sweetening agents. For subgingival gels used for delivery of actives into the periodontal pockets or around the periodontal pockets, a "subgingival gel carrier" is chosen as disclosed in, e.g. U.S. Pat. Nos. 5,198,220 and 5,242,910 both to Damani. Carriers suitable for the preparation of compositions of the present disclosure are well known in the art. Their selection will depend on secondary considerations like taste, cost, and shelf stability, and the like.

Further suitable types of orally acceptable carrier materials or excipients are listed in WO2010/059289, in particular on page 17 - 31, which is incorporated by reference.

The composition of the invention may be added to and incorporated into a product base by known methods. Sufficient may be added to provide a cooling effect. The necessary proportion to provide such an effect will naturally depend on the desired cooling effect, but typical weight proportions being from 0.01 to 0.5%. For examples in an oral application of a compound of the present invention, such as dentifrice, floss, chewing gum, or white strip, the levels of use may be from about 0.00001 % (0.01 ppm) to about 0.1 % (1000 ppm); from about 0.00005% (0.5 ppm) to about 0.1 % (1000 ppm); from about 0.0001 % (1 ppm) to about 0.05% (500 ppm); from about 0.005 % (50 ppm) to about 0.03% (300 ppm); or from about 0.001 % (10 ppm) to about 0.01 % (100 ppm) by weight of the composition. When a compound of the present invention is used in a mouthwash, the level of use may be from about 0.000001 % (10 ppb) to about 0.01 % (100 ppm) or from about 0.0001 % (1 ppm) to about 0.001 % (10 ppm) by weight of the composition. When a compound of the present invention is delivered topically, for example in shampoos and lotions the levels may be from about 0.001 % (10 ppm) to about 0.5% (5000 ppm) by weight of the composition or from about 0.01 % (100 ppm) to about 0.4% (4000 ppm) by weight of the composition. These are general guidelines, not rigid boundaries, and formulators seeking particular effects may find it possible or even desirable to formulate outside them.

The composition and methods are now further described with reference to the following non-limiting examples, which describe particular embodiments.

### EXAMPLES

### Example 1: 2-(methylthio)-1-(2-(5-(p-tolyl)imidazol-2-yl)piperidin-1-yl)propan-1-one

Example 1a: *tert*-butyl-2-(1H-imidazol-2-yl)piperidine-1-carboxylate: To a solution of *tert*-butyl-2-formylpiperidine-1-carboxylate (9.5 g, 35.6 mmol) and glyoxal solution (40% in water, 25.9 g, 178 mmol) in methanol (100 mL) were added dropwise ammonia solution (25 % in water, 17.0 g, 249 mmol) at 0 °C. The solution was allowed to warm up to rt. (room temperature) and stirred at rt. for 16 h. Then the solution was concentrated under reduced pressure, and the result residue was extracted with ethyl acetate (100mL*3). Any precipitate was removed by filtration, and the organic phase was washed with saturated aqueous NaHCO₃ solution (100 mL) and brine (100 mL). The solution was then concentrated under reduced pressure to give *tert*-butyl-2-(1*H-*imidazol-2-yl)piperidine-1-carboxylate (5.2 g, yield: 58 %) as white solid. GC/MS (EI): *m*/*z* (%): 251 (3) [M⁺], 195 (4), 178 (10), 150 (20), 134 (13), 122 (5), 95 (100), 82 (10), 57 (21).

Example 1b: *tert*-butyl-2-(4,5-dibromo-1*H*-imidazol-2-yl)piperidine-1-carboxylate: N-bromosuccinimide (7.4 g, 41.8 mmol) was added portionwise to a solution of tert-butyl-2-(1H-imidazol-2-yl)piperidine-1-carboxylate (5.0 g, 19.9 mmol) in dichloromethane (100 mL) over 10 min at 0 °C. The mixture was stirred at 0 °C for another 2 h and then concentrated by rotary evaporate. The residue was dissolved in ethyl acetate (250 mL), washed with water (100 mL*2) and brine (100 mL), dried with MgSO₄, and concentrated to get a very brown residue. The residue was recrystallized by dichloromethane/hexanes (1 : 1) to get *tert*-butyl-2-(4,5-dibromo-1*H*-imidazol-2-yl)piperidine-1-carboxylate (6.0 g, yield: 74 %) as white solid. GC/MS (EI): *m*/*z* (%): 411 (2) [M⁺], 409 (4) [M⁺], 407 (2) [M⁺], 355 (6), 353 (12), 351 (6), 311 (11), 309 (22), 307 (11), 294 (11), 292 (22), 290 (11), 255 (50), 253 (100), 251 (50), 242 (12), 240 (24), 238 (12), 148 (9), 57 (50).

Example 1c: *tert*-butyl-2-(5-bromo-1*H*-imidazol-2-yl)piperidine-1-carboxylate: A suspension of the *tert*-butyl-2-(4,5-dibromo-1*H*-imidazol-2-yl)piperidine-1-carboxylate (34.0 g, 90%, 74.8 mmol) and Na₂SO₃ (94g, 748 mmol) in ethanol (300 mL) and water (300 mL) was refluxed overnight. Then cool down and concentrated. The residue was partitioned between CH₂Cl₂ (200 mL) and H₂O (200 mL). The aqueous layer was extracted with ethyl acetate (200 mL*3). The combined organic layers were washed with brine (200 mL), dried with Na₂SO₄, filtered and evaporated. The residue was purified by column chromatography on silica gel to give *tert*-butyl-2-(5-bromo-1*H-*imidazol-2-yl)piperidine-1-carboxylate (23.0 g, yield: 93 %) as white solid. GC/MS (EI): *m*/*z* (%): 329 (3) [M⁺], 331 (3) [M⁺], 275 (10), 273 (10), 258 (9), 256 (9), 230 (20), 228 (20), 214 (26), 212 (26). 175 (100), 173 (100), 162 (9), 160 (9), 93 (8), 57 (44).

Example 1d: tert-butyl 2-(5-(p-tolyl)-1*H*-imidazol-2-yl)piperidine-1-carboxylate: A pressure vessel was charged with tert-butyl 2-(5-bromoimidazol-2-yl)piperidine-1-carboxylate (400 mg, 1.211 mmol), p-tolylboronic acid (181 mg, 1.332 mmol, 1.1 equiv.), sodium carbonate (257 mg, 2.42 mmol, 2 equiv.), 1,1'-bis(diphenylphosphino)-ferrocene-palladium(II)dichloride dichloromethane complex (49 mg, 0.061 mmol, 0.05 equiv.), tetrahydrofuran (5 mL) and water (1 mL). The mixture was degassed by purging with nitrogen and the vessel was sealed. The mixture was stirred and heated to 100°C overnight. The resulting mixture was cooled to 0°C, the vessel was opened and the contents poured into aq. sat. NaHCO₃ solution (50 mL), extracted with EtOAc (2x 50 mL), washed with water (50 mL) and brine (50 mL), dried over MgSO₄ and concentrated under reduced pressure. The crude material was purified by silica gel flash column chromatography eluting with a gradient of EtOAc in Heptane to give tert-butyl 2-(5-(p-tolyl)imidazol-2-yl)piperidine-1-carboxylate (314 mg, 0.920 mmol, 76% yield) as a white solid. MS (El, 70 eV): 341 (4, [M]+•), 285 (11), 268 (3), 240 (30), 185 (100), 172 (16), 91 (6), 57 (99). 1H NMR (DMSO-d6, 400MHz, mixture of rotamers and tautomers): *δ* 11.66-12.09 (m, 1H), 7.49-7.70 (m, 2H), 7.20-7.48 (m, 1H), 7.08-7.23 (m, 2H), 5.34 - 5.23 (m, 1H), 3.89 (br d, J=12.1 Hz, 1H), 3.05 (br t, J=10.9 Hz, 1H), 2.28 (s, 3H), 2.18-2.25 (m, 1H), 1.65-1.78 (m, 1H), 1.22-1.63 (m, 13H) ppm. ¹³C NMR (75 MHz, DMSO, mixture of tautomers) δ 155.1 (q), 147.5 (q), 140.2 (q), 135.3 (q), 132.7 (q), 129.4 (t), 124.6 (t), 112.5 (t), 79.3 (q), 63.3 (d), 49.7 (t), 41.2 (d), 28.5 (s), 28.4 (d), 26.8 (d), 25.3 (d), 21.2 (s), 19.9 (d) ppm.

Example 1e: 2-(5-(p-tolyl)-1*H*-imidazol-2-yl)piperidine: A solution of tert-butyl 2-(5-(p-tolyl)imidazol-2-yl)piperidine-1-carboxylate (304 mg, 0.890 mmol) in dichloromethane (3 mL) was treated dropwise at 5°C with trifluoroacetic acid (0.549 mL, 7.12 mmol, 8 equiv.) and the resulting mixture stirred at room temperature for 2 hours or until complete consumption of the starting material. The mixture was poured into iced water (30 mL) and the pH made basic by the addition of aqueous 1M NaOH solution. The mixture was then extracted with dichloromethane (3x 20 mL), dried over MgSO₄ and concentrated under reduced pressure to give 2-(5-(p-tolyl)imidazol-2-yl)piperidine (160 mg, 0.664 mmol, 74% yield) as a pale yellow oil which was used in the next step without further purification. MS (El, 70 eV): 241 (6, [M]+•), 185 (100), 172 (13), 158 (8), 91 (3), 84 (4). 1H NMR (CHLOROFORM-d, 400MHz): δ 8.67-8.89 (br s, 1H), 7.50 (d, J=8.1 Hz, 2H), 7.19 (d, J=7.8 Hz, 2H), 7.13 (s, 1H), 4.14 (dd, J=12.3, 3.1 Hz, 1H), 3.30 (br d, J=12.7 Hz, 1H), 2.71-2.84 (m, 1H), 2.37 (s, 3H), 2.15-2.27 (m, 1H), 2.01 (br dd, J=14.4, 3.2 Hz, 1H), 1.90 (br d, J=13.4 Hz, 1H), 1.67-1.77 (m, 2H), 1.32-1.46 ppm (m, 1H).

Example 1f: 2-(methylthio)-1-(2-(5-(p-tolyl)imidazol-2-yl)piperidin-1-yl)propan-1-one: To a solution of 2-(5-(p-tolyl)imidazol-2-yl)piperidine (0.88 mmol) in Dichloromethane (DCM) (2 mL) was added Hydroxybenzotriazole (HOBt) (1.056 mmol, 1.2 equiv.) and 3-(((ethylimino)methylene)amino)-N,N-dimethylpropan-1-amine hydrochloride (EDCI) (1.056 mmol, 1.2 equiv.) at 0-5 °C and the mixture was stirred at room temperature for 0.5 h. The mixture was then treated with 2-(methylthio)propanoic acid (0.968 mmol, 1.1 equiv.) and N,N-diisopropylethylamine (DIPEA) (0.88 mmol, 1 equiv.) and the resulting mixture was stirred at rt. for 16 h. The mixture was filtered and solvent was removed and the crude purified by silica gel chromatography (gradient of EtOAc in Heptane) to furnish 2-(methylthio)-1-(2-(5-(p-tolyl)imidazol-2-yl)piperidin-1-yl)propan-1-one as a white solid.

MS (El, 70 eV): 343 (2, [M]+•), 241 (17), 240 (100), 213 (13), 185 (18), 184 (9), 75 (55), 56 (11), 55 (9), 47 (10), 41 (11). ¹H NMR (400 MHz, DMSO-d₆, mixture of stereoisomers and tautomers) *δ* 12.07, 11.99, 11.95, 11.76 (brs, 1H), 7.72 - 7.60 (m, 2H), 7.59 - 7.42 (m, 1H), 7.26 - 7.08 (m, 2H), 5.75 - 5.39 (m, 1H), 4.49 - 3.00 (m, 3H), 2.71 - 2.15 (m, 1H), 2.30 (s, 3H), 2.07 - 1.96 (m, 3H), 1.94 - 1.48 (m, 5H), 1.43 - 1.34 (m, 3H) ppm. ¹³C NMR (101 MHz, DMSO-d₆, mixture of stereoisomers and tautomers) *δ* 170.2 (q), 170.2 (q), 170.0 (q), 147.0 (q), 146.8 (q), 146.7 (q), 140.4 (q), 140.3 (q), 139.9 (q), 135.3 (q), 135.2 (q), 135.1 (q), 132.7 (q), 132.6 (q), 132.6 (q), 129.7 (t), 129.3 (t), 124.6 (t), 113.0 (t), 112.6 (t), 112.5 (t), 51.5 (t), 47.3 (t), 47.0 (t), 43.1 (d), 43.0 (d), 38.8 (d), 38.0 (t), 37.6 (t), 37.3 (t), 28.8 (d), 28.6 (d), 28.1 (d), 27.9 (d), 26.1 (d), 25.7 (d), 25.4 (d), 21.2 (s), 20.3 (d), 20.1 (d), 18.3 (s), 18.0 (s), 17.8 (s), 11.9 (s), 11.7 (s), 11.6 (s) ppm.

### Example 2: 2,2-dimethyl-1-(2-(5-(p-tolyl)-1H-imidazol-2-yl)piperidin-1-yl)but-3-en-1-one

Following the general procedure described in Example 1f: 2,2-dimethylbut-3-enoic acid (170 mg, 1.492 mmol), HOBt (228 mg, 1.492 mmol), 3-(((ethylimino)methylene)amino)-*N*,*N*-dimethylpropan-1-amine (232 mg, 1.492 mmol), 2-(5-(*p*-tolyl)-1*H*-imidazol-2-yl)piperidine (300 mg, 1.243 mmol) and DIPEA (0.391 ml, 2.238 mmol) in dichloromethane (30 mL) were reacted to give the title product (255 mg, yield: 68 %) as white solid.

GC/MS (EI): *m*/*z* (%): 337 (1) [*M*⁺], 322 (3), 268 (10), 240 (89), 213 (6), 197 (6), 172 (100), 117(8), 69 (8). ¹H NMR (300 MHz, DMSO-*d₆*, mixture of tautomers) *δ* 12.04 - 11.70 (m, 1H), 7.83 - 7.51 (m, 2H), 7.51 - 7.37 (m, 1H), 7.32 - 7.00 (m, 2H), 6.23 - 6.10 (m, 1H), 5.81 - 5.34 (m, 1H), 5.27 - 4.80 (m, 2H), 4.60 - 2.96 (m, 2H), 2.43 - 2.17 (m, 4H), 1.87 - 1.43 (m, 5H), 1.40 - 1.19 (m, 6H). ¹³C NMR (75 MHz, DMSO-*d₆*, mixture of tautomers) *δ* 174.1 (q), 147.0 (q), 144.4 (t), 139.9 (q), 135.2 (q), 132.7 (q), 129.7 (t), 129.3 (t), 124.5 (t), 112.9 (t), 112.5 (d), 52.7 (t), 47.6 (t), 45.1 (s), 43.9 (d), 28.0 (d), 27.4 (s), 27.1 (s), 25.3 (d), 21.2 (s), 20.2 (d) ppm.

### Example 3: 2-methyl-1-(2-(5-(p-tolyl)-1H-imidazol-2-yl)piperidin-1-yl)butan-1-one

Example 3a: 2-methyl-1-(2-(5-(*p*-tolyl)-1*H*-imidazol-2-yl)piperidin-1-yl)but-3-en-1-one: To a solution of 2-methylbut-3-enoic acid (0.597 g, 5.97 mmol) in dichloromethane (100 mL) was added HOBt (0.914 g, 5.97 mmol) and 3-(((ethylimino)methylene)amino)-*N*,*N-*dimethylpropan-1-amine (0.926 g, 5.97 mmol) at 0~5 °C and the mixture was stirred at room temperature for 0.5 h. Then 2-(5-(*p*-tolyl)-1*H*-imidazol-2-yl)piperidine (1.2 g, 4.97 mmol) and DIPEA (1.563 ml, 8.95 mmol) was added and the mixture was stirred at rt. for 16 h. The suspension was filtered and solvent was removed and the crude product was purified by silica gel chromatography (hexane : MTBE = 3 : 1) to give 2-methyl-1-(2-(5-(*p*-tolyl)-1*H*-imidazol-2-yl)piperidin-1-yl)but-3-en-1-one (808 mg, yield: 50 %) as white solid.

GC/MS (El, mixture of stereoisomers, ratio 1:2): isomer 1: *m*/*z* (%):323 (2) [*M*⁺], 268 (5), 240 (100), 172 (95), 117 (7), 84 (3), 55 (11). isomer 2: *m*/*z* (%):323 (2) [*M*⁺], 268 (5), 240 (100), 211 (6), 172 (81), 117 (7), 84 (2), 55 (10). ¹H NMR (300 MHz, DMSO-d₆, mixture of stereoisomers and tautomers) *δ* 12.06, 11.81 (brs, 1H), 7.69 - 7.62 (m, 2H), 7.49 (s, 1H), 7.15 (d, *J* = 7.3 Hz, 2H), 6.12 - 5.70 (m, 2H), 5.47 - 4.81 (m, 2H), 4.63 - 2.90 (m, 3H), 2.81 - 2.35 (m, 1H), 2.29 (s, 3H), 1.78 - 1.29 (m, 5H), 1.17 (t, *J* = 5.6 Hz, 3H). ¹³C NMR (75 MHz, DMSO-*d₆*, mixture of stereoisomers and tautomers) *δ* 172.7 (q), 172.5 (q), 172.3 (q), 147.1 (q), 146.7 (q), 140.3 (q), 139.8 (q), 139.6 (t), 139.1 (t), 135.3 (q), 132.7 (q), 129.4 (t), 124.6 (t), 115.4 (d), 115.3 (d), 113.0 (t), 112.6 (t), 51.7 (t), 51.3 (t), 47.0 (t), 46.8 (t), 42.8 (d), 42.6 (d), 40.2 (t), 39.3 (t), 38.6 (d), 28.4 (d), 28.1 (d), 26.1 (d), 25.8 (d), 25.3 (d), 21.2 (s), 20.3 (d), 20.1 (d), 18.4 (s), 18.2 (s), 18.0 (s) ppm. Example 3b: 2-methyl-1-(2-(5-(p-tolyl)-1H-imidazol-2-yl)piperidin-1-yl)butan-1-one: 2-methyl-1-(2-(5-(*p*-tolyl)-1*H*-imidazol-2-yl)piperidin-1-yl)but-3-en-1-one (350 mg, 1.082 mmol) was hydrogenated, catalysed by 10% Pd/C (216 mg, 0.216 mmol) in ethyl acetate (20 ml) under hydrogen atmosphere overnight. The mixture was then purged with nitrogen, filtered over celite and evaporated to give a crude material which was purified by flash column chromatography (hexane : MTBE = 3 : 1) to give the title product (290 mg, yield: 82 %) as white solid.

GC/MS (EI): *m*/*z* (%): 325 (10) [*M*⁺], 268 (2), 240 (100), 224 (3), 185 (10), 159(2), 142 (1), 84 (2), 57 (4). ¹H NMR (300 MHz, DMSO-*d₆*, mixture of stereoisomers and tautomers) δ 12.05 - 11.72 (m, 1H), 7.73 - 7.53 (m, 2H), 7.51 - 7.40 (m, 1H), 7.27 - 7.03 (m, 2H), 5.81 - 5.28 (m, 1H), 4.61 - 3.16 (m, 2H), 2.87 - 2.58 (m, 1H), 2.38 - 2.19 (m, 4H), 1.86 - 1.49 (m, 5H), 1.46 - 1.23 (m, 2H), 1.09 - 0.95 (m, 3H), 0.94 - 0.80 (m, 3H). ¹³C NMR (75 MHz, DMSO-*d₆*, mixture of stereoisomers and tautomers) *δ* 175.4 (q), 175.2 (q), 147.3 (q), 147.2 (q), 140.5 (q), 140.1 (q), 135.2 (q), 132.7 (q), 129.7 (t), 129.3 (t), 124.6 (t), 112.9 (t), 112.6 (t), 51.5 (t), 46.9 (t), 46.8 (t), 42.6 (d), 38.6 (d), 36.8 (t), 36.5 (t), 36.1 (t), 29.2 (d), 28.9 (d), 28.4 (d), 27.2 (d), 26.9 (d), 26.2 (d), 25.4 (d), 25.2 (d), 21.2 (s), 20.3 (d), 18.4 (s), 17.7 (s), 17.3 (s), 12.2 (s), 12.0 (s), 11.9 (s) ppm.

### Example 4: 2-methyl-2-(methylthio)-1-(2-(5-(p-tolyl)-1H-imidazol-2-yl)piperidin-1-yl)propan-1-one

Following the general procedure described in Example 1f: 2-methyl-2-(methylthio)propanoic acid (0.267 g, 1.989 mmol), HOBt (305 mg, 1.989 mmol), 3-(((ethylimino)methylene)amino)-*N*,*N*-dimethylpropan-1-amine (309 mg, 1.989 mmol), 2-(5-(*p*-tolyl)-1*H*-imidazol-2-yl)piperidine (400 mg, 1.657 mmol) and DIPEA (0.521 ml, 2.980 mmol) in dichloromethane (30 mL) were reacted to give the title product (287 mg, yield: 48 %) as white solid.

GC/MS (EI): *m*/*z* (%): 357 (5) [*M*⁺], 342(7), 268 (11), 240 (100), 185 (11), 159 (5), 117 (4), 89 (10). ¹H NMR (300 MHz, DMSO-*d₆*, mixture of tautomers) *δ* 12.06, 11.82 (brs, 1H), 7.80 - 7.54 (m, 2H), 7.50 (s, 1H), 7.33 - 7.08 (m, 2H), 6.15 - 5.89 (m, 1H), 4.92 - 2.73 (m, 2H), 2.47 - 2.23 (m, 4H), 2.19 - 2.03 (m, 3H), 1.86 - 1.35 (m, 11H). ¹³C NMR (75 MHz, DMSO-*d₆*, mixture of tautomers) *δ* 171.1 (q), 170.8 (q), 148.3 (q), 146.8 (q), 140.1 (q), 136.1 (q), 135.2 (q), 132.6 (q), 131.6 (q), 129.7 (t), 129.7 (t), 129.3 (t), 124.6 (t), 112.8 (t), 53.5 (t), 48.4 (t), 47.6 (q), 44.4 (d), 29.3 (d), 28.6 (d), 27.6 (s), 25.8 (d), 21.2 (s), 20.2 (d), 12.9 (s) ppm.

### Example A : solubility of non-natural cooling compounds

Into a 20ml vial 0.4 g of the respective non-natural cooling compound as indicated in Table 1 below have been added followed by the addition of 1.6 g lactic acid. The vial was warmed to about 50°C with gentle agitation for 1 hour. If the respective non-natural cooling compound hasn't dissolved, 2 g lactic acid was added (resulting in a composition comprising 10 weight % of a non-natural cooling compound), and again gentle agitated for an additional hour at about 50°C. If the non-natural cooling compound still hasn't dissolved, 4 g of lactic acid was added (resulting in a composition comprising 5 weight % of anon-natural cooling compound), and again gentle agitated for an additional hour at about 50°C.

If the non-natural cooling compound still has not dissolved, the procedure above has been repeated, starting with 0.05g of the non-natural cooling compound and 0.95 g of lactic acid. If still not dissolved, 1g lactic acid was added (resulting in a composition comprising 2.5 weight % of a non-natural cooling compound). If still not dissolved further solvent was added. The results are shown in Table 1 below.

If 0.4 g the non-natural cooling compound dissolved in 1.6g lactic acid, the procedure above has been repeated, starting with 1 g of the non-natural cooling compound and 1g lactic acid. If the respective non-natural cooling compound hasn't dissolved, 0.5 g lactic acid was added (resulting in a composition comprising 40 weight % of a non-natural cooling compound), and again gentle agitated for an additional hour at about 50°C. If the non-natural cooling compound still hasn't dissolved, 0.83 g of lactic acid was added (resulting in a composition comprising 30 weight % of anon-natural cooling compound), and again gentle agitated for an additional hour at about 50°C.

**Table 1: Measure of the solubility of non-natural cooling compounds in lactic acid**

| **Non-natural cooling compound** | **Soluble solution** |
|---|---|
| 2-methyl-1-(2-(5-(p-tolyl)-1H-imidazol-2-yl)piperidin-1-yl)butan-1-one | 50 wt % |
| N-(1H-pyrazol-3-yl)-N-(thiophen-2-ylmethyl)-2-(p-tolyloxy)acetamide | 30 wt % |
| 2-(methylthio)-1-(2-(5-(p-tolyl)-1H-imidazol-2-yl)piperidin-1-yl)propan-1-one | 50 wt % |
| 2-methyl-2-(methylthio)-1-(2-(5-(p-tolyl)-1H-imidazol-2-yl)piperidin-1-yl)propan-1-one | 40 wt % |
| 2,2-dimethyl-1-(2-(5-(p-tolyl)-1H-imidazol-2-yl)piperidin-1-yl)but-3-en-1-one | 30 wt % |
| 2-isopropyl-5-methyl-N-(2-(pyridin-2-yl)ethyl)cyclohexane-1-carboxamide (Evercool^{®} 190) | 20 wt % |
| 2-(4-ethylphenoxy)-N-(1H-pyrazol-3-yl)-N-(thiophen-2-ylmethyl)acetamide | 5 wt % |
| N-Ethyl-p-menthane-3-carboxamide (WS 3) | 10 wt% |
| ethyl (2-isopropyl-5-methylcyclohexane-1-carbonyl)glycinate (WS 5) | 20 wt% |
| 2-isopropyl-N,2,3-trimethylbutanamide (WS 23) | 20 wt% |
| N-(4-(2-amino-2-oxoethyl)phenyl)-2-isopropyl-5-methylcyclohexane-1-carboxamide) | 2.5 wt% |
| 2-isopropyl-5-methylcyclohexyl 2-hydroxypropanoate | 20 wt% |
| N-(2-hydroxy-2-phenylethyl)-2-isopropyl-5,5-dimethylcyclohexane-1-carboxamide | 5 wt% |

### Example B: Preparation of a flavor formulation

The flavoring compositions were prepared by admixing the following ingredients:

| Ingredient | parts by weight |
|---|---|
| CLOVE TERPENELESS | 0.3 |
| ETHYL MALTOL | 0.1 |
| 20 wt% solution of Cooling compound* in lactic acid | 1.0 |
| GINGER OLEORESIN | 0.1 |
| LEMON Oil | 0.5 |
| MENTHOL LAEVO EXTRA | 58.0 |
| ORANGE Oil | 0.5 |
| PEPPERMINT Oil | 35.0 |
| PROPYLENE GLYCOL | 0.4 |
| SPEARMINT Oil | 4.0 |
| VANILLIN | 0.1 |
| | 100.0 |

| Ingredient | parts by weight |
|---|---|
| CLOVE TERPENELESS | 1.6 |
| ETHYL MALTOL | 0.1 |
| EUCALYPTUS Oil | 3.0 |
| EUGENOL | 6.0 |
| 20 wt% solution of Cooling compound* in lactic acid | 0.9 |
| MENTHOL LAEVO | 56.0 |
| MENTHOL RACEMIC | 8.0 |
| METHYL SALICYLATE | 18.2 |
| PEPPERMINT Oil | 6.0 |
| TEA TREE PURE | 0.1 |
| TOSCANOL** | 0.1 |
| | 100.00 |

| Ingredient | parts by weight |
|---|---|
| CARVONE LAEVO | 12.0 |
| ETHYL MALTOL | 0.1 |
| 20 wt% solution of Cooling compound* in lactic acid | 1.12 |
| MENTHOL LAEVO | 42.7 |
| PEPPERMINT Oil | 16.0 |
| SPEARMINT Oil | 28.0 |
| VANILLA extract | 0.08 |
| | 100.0 |

| | |
|---|---|
| *: 2-methyl-1-(2-(5-(*p*-tolyl)-1*H*-imidazol-2-yl)piperidin-1-yl)butan-1-one **: 1-(cyclopropylmethyl)-4-methoxybenzene; origin: Givaudan SA, Vernier, Switzerland | |

The flavoring compositions may be added to an oral care product (e.g. mouth wash) or to confectionary (e.g. chewing gum). If added to a chewing gum base however, higher amounts of lactic acid influence the texture of the gum base, resulting in the hardening of a chewing gum base. On the other hand, the addition of higher amounts of lactic acid to hard candy bases may result in the softening of such bases.

## Claims

1. A liquid composition comprising, consisting essentially of, or consisting of
a) a non-natural cooling compound, and
b) lactic acid, and
wherein the non-natural cooling compound is selected from the group consisting of 2-methyl-1-(2-(5-(p-tolyl)-1H-imidazol-2-yl)piperidin-1-yl)butan-1-one (including (2S)-2-methyl-1-(2-(5-(p-tolyl)-1H-imidazol-2-yl)piperidin-1-yl)butan-1-one, and (2R)-2-methyl-1-(2-(5-(p-tolyl)-1H-imidazol-2-yl)piperidin-1-yl)butan-1-one), 2-(methylthio)-1-(2-(5-(p-tolyl)-1H-imidazol-2-yl)piperidin-1-yl)propan-1-one, 2-methyl-2-(methylthio)-1-(2-(5-(p-tolyl)-1H-imidazol-2-yl)piperidin-1-yl)propan-1-one, 2,2-dimethyl-1-(2-(5-(p-tolyl)-1H-imidazol-2-yl)piperidin-1-yl)but-3-en-1-one, N-(1H-pyrazol-3-yl)-N-(thiophen-2-ylmethyl)-2-(p-tolyloxy)acetamide, 2-(4-ethylphenoxy)-N-(1H-pyrazol-3-yl)-N-(thiophen-2-ylmethyl)acetamide, N-Ethyl-p-menthane-3-carboxamide, ethyl (2-isopropyl-5-methylcyclohexane-1-carbonyl)glycinate, 2-isopropyl-N,2,3-trimethylbutanamide, N-(4-(2-amino-2-oxoethyl)phenyl)-2-isopropyl-5-methylcyclohexane-1-carboxamide (including *rac*-(1R,2S,5R)-N-(4-(2-amino-2-oxoethyl)phenyl)-2-isopropyl-5-methylcyclohexane-1-carboxamide), 2-isopropyl-5-methylcyclohexyl 2-hydroxypropanoate, N-(2-hydroxy-2-phenylethyl)-2-isopropyl-5,5-dimethylcyclohexane-1-carboxamide, and mixtures thereof.

2. A liquid composition according to claim 1, wherein the composition comprises at least 1 weight % of at least one non-natural cooling compound, relative to the total weight of the composition.

3. A fragrance or flavour formulation comprising a composition as defined in any proceeding claim, further comprising at least one active selected from the group consisting of flavour and fragrance, and optionally comprising sweetening agent.

4. A fragranced or flavoured product, comprising a product base and a cooling effect-providing proportion of a composition according to any one of claims 1 - 2, or a formulation according to claim 3.

5. A method of incorporating at least one non-natural cooling compound in a fragranced or flavoured product, comprising the step of blending (a) at least one non-natural cooling compound with lactic acid, and the addition of the resulting composition to the product, wherein the non-natural cooling compound is selected from the group consisting of 2-methyl-1-(2-(5-(p-tolyl)-1H-imidazol-2-yl)piperidin-1-yl)butan-1-one (including (2S)-2-methyl-1-(2-(5-(p-tolyl)-1H-imidazol-2-yl)piperidin-1-yl)butan-1-one, and (2R)-2-methyl-1-(2-(5-(p-tolyl)-1H-imidazol-2-yl)piperidin-1-yl)butan-1-one), 2-(methylthio)-1-(2-(5-(p-tolyl)-1H-imidazol-2-yl)piperidin-1-yl)propan-1-one, 2-methyl-2-(methylthio)-1-(2-(5-(p-tolyl)-1H-imidazol-2-yl)piperidin-1-yl)propan-1-one, 2,2-dimethyl-1-(2-(5-(p-tolyl)-1H-imidazol-2-yl)piperidin-1-yl)but-3-en-1-one, N-(1H-pyrazol-3-yl)-N-(thiophen-2-ylmethyl)-2-(p-tolyloxy)acetamide, 2-(4-ethylphenoxy)-N-(1H-pyrazol-3-yl)-N-(thiophen-2-ylmethyl)acetamide, N-Ethyl-p-menthane-3-carboxamide, ethyl (2-isopropyl-5-methylcyclohexane-1-carbonyl)glycinate, 2-isopropyl-N,2,3-trimethylbutanamide, N-(4-(2-amino-2-oxoethyl)phenyl)-2-isopropyl-5-methylcyclohexane-1-carboxamide (including *rac*-(1R,2S,5R)-N-(4-(2-amino-2-oxoethyl)phenyl)-2-isopropyl-5-methylcyclohexane-1-carboxamide), 2-isopropyl-5-methylcyclohexyl 2-hydroxypropanoate, N-(2-hydroxy-2-phenylethyl)-2-isopropyl-5,5-dimethylcyclohexane-1-carboxamide, and mixtures thereof.

6. A method of providing to a product a cooling sensation for the skin or the mucous membrane, comprising the addition to a product base of a cooling effect-providing proportion of a composition according to any one of claims 1 to 2, or a formulation according to claim 3.

## Patentansprüche

1. Flüssige Zusammensetzung umfassend, im Wesentlichen bestehend aus oder bestehend aus
a) einer nichtnatürlichen kühlenden Verbindung und
b) Milchsäure, und
wobei die nichtnatürliche kühlende Verbindung aus der Gruppe bestehend aus 2-Methyl-1-(2-(5-(p-tolyl)-1H-imidazol-2-yl)piperidin-1-yl)butan-1-on (einschließlich (2S)-2-Methyl-1-(2-(5-(p-tolyl)-1H-imidazol-2-yl)piperidin-1-yl)butan-1-on und (2R)-2-Methyl-1-(2-(5-(p-tolyl)-1H-imidazol-2-yl)piperidin-1-yl)butan-1-on), 2-(Methylthio)-1-(2-(5-(p-tolyl)-1H-imidazol-2-yl)piperidin-1-yl)propan-1-on, 2-Methyl-2-(methylthio)-1-(2-(5-(p-tolyl)-1H-imidazol-2-yl)piperidin-1-yl)propan-1-on, 2,2-Dimethyl-1-(2-(5-(p-tolyl)-1H-imidazol-2-yl)piperidin-1-yl)but-3-en-1-on, N-(1H-Pyrazol-3-yl)-N-(thiophen-2-ylmethyl)-2-(p-tolyloxy)acetamid, 2-(4-Ethylphenoxy)-N-(1H-pyrazol-3-yl)-N-(thiophen-2-ylmethyl)acetamid, N-Ethyl-p-menthan-3-carboxamid, (2-Isopropyl-5-methylcyclohexan-1-carbonyl)glycinethylester, 2-Isopropyl-N,2,3-trimethylbutanamid, N-(4-(2-Amino-2-oxoethyl)phenyl)-2-isopropyl-5-methylcyclohexan-1-carboxamid (einschließlich *rac*-(1R,2S,5R)-N-(4-(2-Amino-2-oxoethyl)phenyl)-2-isopropyl-5-methylcyclohexan-1-carboxamid), 2-Hydroxypropansäure-2-isopropyl-5-methylcyclohexylester, N-(2-Hydroxy-2-phenylethyl)-2-isopropyl-5,5-dimethylcyclohexan-1-carboxamid und Mischungen davon ausgewählt ist.

2. Flüssige Zusammensetzung nach Anspruch 1, wobei die Zusammensetzung mindestens 1 Gew.-% mindestens einer nichtnatürlichen kühlenden Verbindung, bezogen auf das Gesamtgewicht der Zusammensetzung, umfasst.

3. Duftstoff- oder Geschmacksstoffformulierung, umfassend eine Zusammensetzung gemäß einem der vorhergehenden Ansprüche, ferner umfassend mindestens eine aktive Substanz, die aus der Gruppe bestehend aus Geschmacksstoff und Duftstoff ausgewählt ist, und gegebenenfalls Süßungsmittel.

4. Duftstoff- oder geschmacksstoffhaltiges Produkt, umfassend eine Produktbasis und einen eine kühlende Wirkung bereitstellenden Anteil einer Zusammensetzung nach einem der Ansprüche 1 - 2 oder einer Formulierung nach Anspruch 3.

5. Verfahren zur Einarbeitung mindestens einer nichtnatürlichen kühlenden Verbindung in ein duftstoff- oder geschmacksstoffhaltiges Produkt, umfassend das Mischen mindestens einer nichtnatürlichen kühlenden Verbindung mit Milchsäure und das Zugeben der resultierenden Zusammensetzung zu dem Produkt, wobei die nichtnatürliche kühlende Verbindung aus der Gruppe bestehend aus 2-Methyl-1-(2-(5-(p-tolyl)-1H-imidazol-2-yl)piperidin-1-yl)butan-1-on (einschließlich (2S)-2-Methyl-1-(2-(5-(p-tolyl)-1H-imidazol-2-yl)piperidin-1-yl)butan-1-on und (2R)-2-Methyl-1-(2-(5-(p-tolyl)-1H-imidazol-2-yl)piperidin-1-yl)butan-1-on), 2-(Methylthio)-1-(2-(5-(p-tolyl)-1H-imidazol-2-yl)piperidin-1-yl)propan-1-on, 2-Methyl-2-(methylthio)-1-(2-(5-(p-tolyl)-1H-imidazol-2-yl)piperidin-1-yl)propan-1-on, 2,2-Dimethyl-1-(2-(5-(p-tolyl)-1H-imidazol-2-yl)piperidin-1-yl)but-3-en-1-on, N-(1H-Pyrazol-3-yl)-N-(thiophen-2-ylmethyl)-2-(p-tolyloxy)acetamid, 2-(4-Ethylphenoxy)-N-(1H-pyrazol-3-yl)-N-(thiophen-2-ylmethyl)acetamid, N-Ethyl-p-menthan-3-carboxamid, (2-Isopropyl-5-methylcyclohexan-1-carbonyl)glycinethylester, 2-Isopropyl-N,2,3-trimethylbutanamid, N-(4-(2-Amino-2-oxoethyl)phenyl)-2-isopropyl-5-methylcyclohexan-1-carboxamid (einschließlich *rac*-(1R,2S,5R)-N-(4-(2-Amino-2-oxoethyl)phenyl)-2-isopropyl-5-methylcyclohexan-1-carboxamid), 2-Hydroxypropansäure-2-isopropyl-5-methylcyclohexylester, N-(2-Hydroxy-2-phenylethyl)-2-isopropyl-5,5-dimethylcyclohexan-1-carboxamid und Mischungen davon ausgewählt wird.

6. Verfahren zum Versehen eines Produkts mit einer kühlenden Empfindung für die Haut oder die Schleimhaut, umfassend das Zugeben eines eine kühlende Wirkung bereitstellenden Anteils einer Zusammensetzung nach einem der Ansprüche 1 bis 2 oder einer Formulierung nach Anspruch 3 zu einer Produktbasis.

## Revendications

1. Composition liquide comprenant, essentiellement constituée, ou constituée
a) d'un composé de refroidissement non naturel, et
b) d'acide lactique, et
dans laquelle le composé de refroidissement non naturel est choisi dans le groupe constitué de 2-méthyl-1-(2-(5-(p-tolyl)-1H-imidazol-2-yl)pipéridin-1-yl)butan-1-one (y compris (2S)-2-méthyl-1-(2-(5-(p-tolyl)-1H-imidazol-2-yl)pipéridin-1-yl)butan-1-one et (2R)-2-méthyl-1-(2-(5-(p-tolyl)-1H-imidazol-2-yl)pipéridin-1-yl)butan-1-one), 2-(méthylthio)-1-(2-(5-(p-tolyl)-1H-imidazol-2-yl)pipéridin-1-yl)propan-1-one, 2-méthyl-2-(méthylthio)-1-(2-(5-(p-tolyl)-1H-imidazol-2-yl)pipéridin-1-yl)propan-1-one, 2,2-diméthyl-1-(2-(5-(p-tolyl)-1H-imidazol-2-yl)pipéridin-1-yl)but-3-én-1-one, N-(1H-pyrazol-3-yl)-N-(thiophén-2-ylméthyl)-2-(p-tolyloxy)acétamide, 2-(4-éthylphénoxy)-N-(1H-pyrazol-3-yl)-N-(thiophén-2-ylméthyl)acétamide, *N*-éthyl-*p-*menthane-3-carboxamide, (2-isopropyl-5-méthylcyclohexane-1-carbonyl)glycinate d'éthyle, 2-isopropyl-N,2,3-triméthylbutanamide, N-(4-(2-amino-2-oxoéthyl)phényl)-2-isopropyl-5-méthylcyclohexane-1-carboxamide (y compris *rac*-(1R,2S,5R)-N-(4-(2-amino-2-oxoéthyl)phényl)-2-isopropyl-5-méthylcyclohexane-1-carboxamide), 2-hydroxypropanoate de 2-isopropyl-5-méthylcyclohexyle, N-(2-hydroxy-2-phényléthyl)-2-isopropyl-5,5-diméthylcyclohexane-1-carboxamide et leurs mélanges.

2. Composition liquide selon la revendication 1, dans laquelle la composition comprend au moins 1 % en poids d'au moins un composé de refroidissement non naturel, par rapport au poids total de la composition.

3. Formulation de fragrance ou d'arôme comprenant une composition telle que définie dans l'une quelconque des revendications précédentes, comprenant en outre au moins un agent actif choisi dans le groupe constitué d'un arôme et d'une fragrance, et comprenant éventuellement un agent édulcorant.

4. Produit fragrancé ou aromatisé, comprenant une base de produit et une proportion fournissant un effet de refroidissement d'une composition selon l'une quelconque des revendications 1 à 2, ou d'une formulation selon la revendication 3.

5. Procédé d'incorporation d'au moins un composé de refroidissement non naturel dans un produit fragrancé ou aromatisé, comprenant l'étape de mélange (a) d'au moins un composé de refroidissement non naturel avec de l'acide lactique, et l'ajout de la composition résultante au produit, le composé de refroidissement non naturel étant choisi dans le groupe constitué de 2-méthyl-1-(2-(5-(p-tolyl)-1H-imidazol-2-yl)pipéridin-1-yl)butan-1-one (y compris (2S)-2-méthyl-1-(2-(5-(p-tolyl)-1H-imidazol-2-yl)pipéridin-1-yl)butan-1-one et (2R)-2-méthyl-1-(2-(5-(p-tolyl)-1H-imidazol-2-yl)pipéridin-1-yl)butan-1-one), 2-(méthylthio)-1-(2-(5-(p-tolyl)-1H-imidazol-2-yl)pipéridin-1-yl)propan-1-one, 2-méthyl-2-(méthylthio)-1-(2-(5-(p-tolyl)-1H-imidazol-2-yl)pipéridin-1-yl)propan-1-one, 2,2-diméthyl-1-(2-(5-(p-tolyl)-1H-imidazol-2-yl)pipéridin-1-yl)but-3-én-1-one, N-(1H-pyrazol-3-yl)-N-(thiophén-2-ylméthyl)-2-(p-tolyloxy)acétamide, 2-(4-éthylphénoxy)-N-(1H-pyrazol-3-yl)-N-(thiophén-2-ylméthyl)acétamide, *N*-éthyl-*p-*menthane-3-carboxamide, (2-isopropyl-5-méthylcyclohexane-1-carbonyl)glycinate d'éthyle, 2-isopropyl-N,2,3-triméthylbutanamide, N-(4-(2-amino-2-oxoéthyl)phényl)-2-isopropyl-5-méthylcyclohexane-1-carboxamide (y compris *rac*-(1R,2S,5R)-N-(4-(2-amino-2-oxoéthyl)phényl)-2-isopropyl-5-méthylcyclohexane-1-carboxamide), 2-hydroxypropanoate de 2-isopropyl-5-méthylcyclohexyle, N-(2-hydroxy-2-phényléthyl)-2-isopropyl-5,5-diméthylcyclohexane-1-carboxamide et leurs mélanges.

6. Procédé pour fournir à un produit une sensation de refroidissement pour la peau ou la membrane muqueuse, comprenant l'ajout à une base de produit d'une proportion fournissant un effet de refroidissement d'une composition selon l'une quelconque des revendications 1 à 2, ou d'une formulation selon la revendication 3.
